Europäisches Patentamt

European Patent Office

Office européen des brevets

(11) Publication number: **0 181 139**
**A2**

# (12) EUROPEAN PATENT APPLICATION

(21) Application number: **85307810.3**

(22) Date of filing: **29.10.85**

(51) Int. Cl.⁴: **A 61 M 1/14**

(30) Priority: **06.11.84 JP 233679/84**

(43) Date of publication of application:
**14.05.86 Bulletin 86/20**

(84) Designated Contracting States:
**BE CH DE FR GB IT LI NL SE**

(71) Applicant: **KANEGAFUCHI KAGAKU KOGYO KABUSHIKI KAISHA**
**2-4 Nakanoshima 3-chome**
**Kita-ku Osaka-shi Osaka-fu(JP)**

(72) Inventor: **Ohnishi, Michikazu**
**1-35-402, 2-chome, Tsurukabuto**
**Nada Kobe Hyogo(JP)**

(72) Inventor: **Takada, Satoshi**
**1-1-3-704, Yoshida**
**Hyogo Kobe(JP)**

(74) Representative: **Laredo, Jack Joseph et al,**
**Elkington and Fife High Holborn House 52/54 High Holborn**
**London, WC1V 6SH(GB)**

(54) Method and apparatus for priming and purging blood treatment apparatus.

(57) In this method for initially priming a blood constituent treatment circuit which includes one or more various blood treatment devices, the steps are included of: switching by a controller one or more conduit switching valves provided at appropriate locations in the blood constituent treating circuit so as to create a local circuit in one part thereof; circulating to the local circuit an initial circulating fluid of physiological saline fluid or an equivalent thereof; and initiating by the controller a completing action according to visual evaluation by the operator. The controller terminates one such set of steps before again performing another such set of steps to create another separate local circuit, the steps being repeatedly performed until substantially all of the air and other foreign matter in the blood treatment devices in the blood constituent treatment circuit has been purged and the blood constituent treatment circuit is substantially full only of the initial circulating fluid. A device is also disclosed for performing the method.

0181139

METHOD AND APPARATUS FOR PRIMING

AND PURGING BLOOD TREATMENT APPARATUS

**BACKGROUND OF THE INVENTION**

The present invention relates to a method and an apparatus for priming a blood treatment apparatus such as one which replaces, removes, cleans, and/or treats blood of a patient, and in particular relates to such a method and apparatus which ensure quick and easy priming of the apparatus in a safe, simple, and reliable fashion.

In the art, there are various machines known for the treatment of blood, typically human blood. Such machines are used for the medical treatment of conditions such as drug intoxication, acute hepatitis, hyperglobulinemia, hyperlipemia, multiple myeloma, myasthenia gravis, rheumatoid arthritis, liver failure, erythematosus, and nephritis, and such machines can make available various treatments for such blood constituents as blood corpuscles, blood plasma, and so on. For the sake of simplicity of explanation, in the following description, explanation will be particularly directed to a method and an apparatus for removing low density lipoprotein from blood plasma, but this is not intended to be limitative of the present invention.

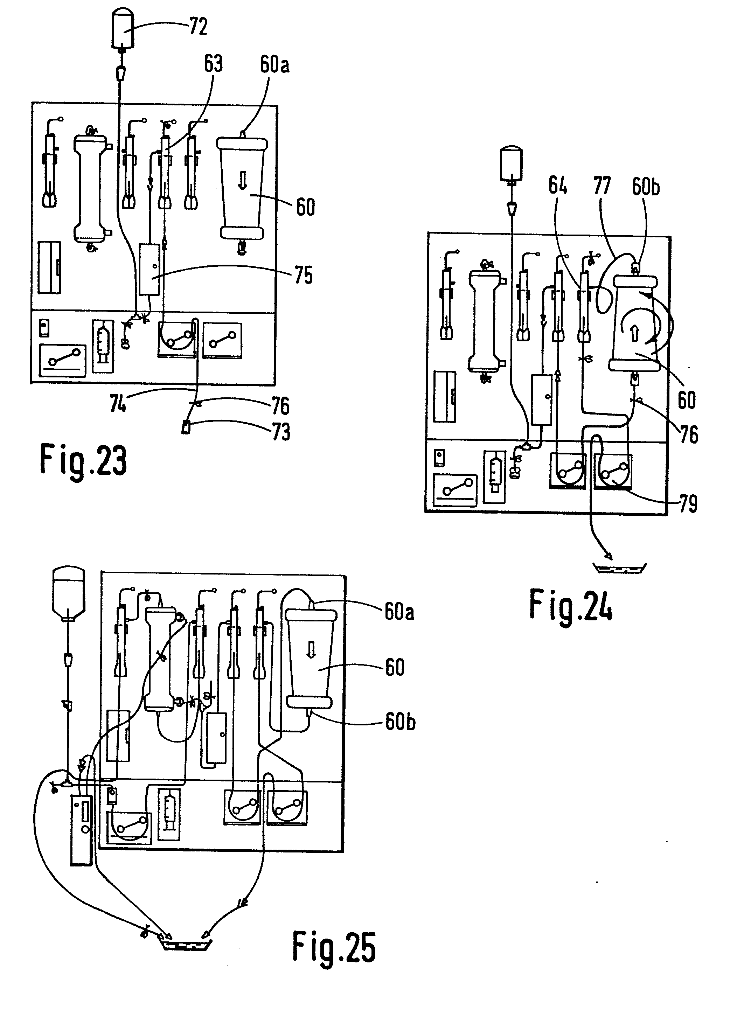

A prior art type such apparatus for performing blood treatment is shown in Fig. 21 of the accompanying drawings in a general perspective view, and this machine, along with its operation, will now be described in order to bring the shortcomings of such prior art into focus. This machine, designated generally by the reference numeral 1, has a setting up panel 2 as its accessible upper front surface, and a control panel 3 at its upper portion.

The setting up panel 2 comprises a substantially level or slightly sloping lower surface portion 2a which is provided with a blood pump 7 with its driving apparatus, a heparin pump 8, a liquid supply pump 9, and various other such per se known devices, and further comprises a substantially upright upper surface portion 2b on which is set up an exemplary circuit for removing low density lipo protein from blood plasma. In fixed positions on a diagram of said circuit shown on said upright upper surface portion 2b are provided holding clamps 4, 5, and 6 for holding various apparatuses such as a blood plasma separator, a blood plasma filterer, drippers, and the like. Further, a stand 10 for hanging objects at a height, a panel heater 11, a load sensor 12 are all attached to the side portion of the blood treatment machine 1.

The control panel 3 is provided in a position at the top of the machine 1 as somewhat inclined from the vertical orientation, and is provided with various types of apparatus for measuring parameters associated with the blood treatment process, to the end of monitoring the ongoing operation of the blood treatment machine. These measuring apparatuses may include, for example, a pressure meter (not particularly identified in the figure) for monitoring and displaying the current value of the pressure in the space at the top portion of a dripper which is attached to the setting up panel 2. Further, the control panel 3 is provided with operating buttons, switches and the like for controlling the various apparatuses which are attached to said setting up panel 2.

Now, when a patient is to be treated, it is necessary to prime the various fluid circuits provided in these various apparatuses which are attached to said setting up panel, in order to ensure proper functioning of all said apparatuses, as well as in order to positively purge and eliminate air and other foreign matter therein, and, as will be seen shortly, this is in the prior art a very laborious, troublesome, and error prone job. Figs. 22 through 25 of the accompanying drawings show an exemplary such priming and purging process for a fluid circuit set up on the Fig. 21 apparatus, said circuit including a blood plasma filterer 60. In the following, a simplified explanation of one part of the initial priming and purging process for this circuit will be given based on these figures.

First, as shown in Fig. 22, the fixed apparatuses, i.e. the blood plasma separator, the blood plasma filterer 60, first through fourth drippers 61, 62, 63, and 64, and so on, are set in position in the various holding clamps 4, 5, 6, and possibly others on the setting up panel 2 of Fig. 21. Next, the ends of fluid supply conduits 65 through 71 are connected to the drippers 61 through 64.

Next, as shown in Fig. 23, a pack 72 of physiological saline solution is suspended from the stand 10 at a height, and said saline pack 72 and the third dripper 63 are connected together via a blood leakage detector 75. When the physiological saline solution begins to flow out of the unconnected end 73 of the fluid supply conduit 74 connected to this third dripper 63, then, as schematically shown in the figure, forceps 76 are applied so as to close off a portion of said fluid supply conduit 74 close to its end, and said end of said fluid supply conduit 74 is fitted to the connection 60a at the upper portion of the blood filterer 60.

Next, as shown in Fig. 24, the blood filterer 60 is inverted, by turning it and the clamp 5 which is holding it around a horizontal axis through an angle of approximately 180°, said clamp 5 being so mounted as to

- 5 -

0181139

be thus rotatable. Thereby, the connection 60a now is located at the bottom of said blood filterer 60, while another connection 60b thereof becomes uppermost. And next this connection 60b of said blood filterer 60 is connected, via a fluid supply conduit 77, to the fourth dripper 64, and this fourth dripper 64 is connected, via a fluid supply conduit 70 on an intermediate position of which forceps 78 are fitted, to the fluid supply pump 79.

Finally, as shown in Fig. 25, the blood filterer 60 is again inverted, so as to return it to its normal orientation, by turning it and the clamp 5 again around a horizontal axis through an angle of approximately 180°, and the forceps are removed. Thereby the priming and purging of this portion of the apparatus are accomplished.

Other parts of this prior art blood treatment machine 1 are primed and purged in manners essentially similar to the above: various fluid supply or receiving conduits are connected and disconnected by hand by the operator to the various apparatuses mounted on the setting up panel 2, and forceps are applied to and are taken off from these conduits as required in order to establish or interrupt communication therethrough. The various fluid circuits through the blood treatment apparatus are all primed and purged in this way, so as to eliminate all of the air initially

0181139

contained in them and so as to cleanse them of foreign matter, and thus the setup work for the apparatus is completed.

According to this prior art priming and purging system, the work is extremely laborious and complicated, because of the repeated connection and disconnection of the various fluid conduits, and it in practice is difficult to perform without constant consultation of an operation manual for the blood treatment machine. Further, since the conduits are often removed from one apparatus and fitted to another apparatus, the danger of contamination by germs and other foreign matter is quite acute. If detected, this means that the entire machine must be dismantled and disinfected and then reconnected and reprimed and purged, thus more than doubling the labor of the cleansing operation. Accordingly, prior art methods and apparatuses for priming and purging a blood treatment apparatus require inordinate amounts of time, even if an extremely skilled person is performing the operation. Moreover, because the connection sequence for the fluid supply conduits is extremely complicated and painstaking, there is a risk of serious mistakes being made, such as mistaken conduit connection, neglect of forceps removal, and, in extreme cases, omission of a step in the initial priming process. Of course, because this blood treatment machine 1 is used for being directly connected with the circulatory system of a human patient and for directly treating the blood of said patient, any error of the above kind that occurs in the course of priming of the machine is extremely dangerous, in that the operation of the machine is directly related to the life of the patient.

SUMMARY OF THE INVENTION

Accordingly, it is the primary object of the present invention to provide a method for priming and purging a blood treatment apparatus, which overcomes the problems detailed above.

It is a further object of the present invention to provide such a method for priming and purging a blood treatment apparatus, which minimizes the amount of connection and disconnection that is required for blood transfer conduits.

It is a further object of the present invention to provide such a method for priming and purging a blood treatment apparatus, which minimizes the amount of forceps placement and removal that are required during operation.

It is a further object of the present invention to provide such a method for priming and purging a blood treatment apparatus, which, while proceeding in a partly automatic fashion, can pause at the end of each step to invite operator checking and feedback.

It is a further object of the present invention to provide such a method for priming and purging a blood treatment apparatus, which is less complicated in operation than the prior art.

It is a further object of the present invention to provide such a method for priming and purging a blood treatment apparatus, which is less troublesome and requires less skill for the operator than in the prior art.

It is a yet further object of the present invention to provide such a method for priming and purging a blood treatment apparatus, which is less prone to the occurrence of dangerous error than has been the case.

It is a yet further object of the present invention to provide an apparatus for priming and purging a blood treatment apparatus, which aids in the achievement of the aforementioned method objects.

According to the method aspect of the present invention, these and other objects are accomplished by a method for initially priming a blood constituent treatment circuit which includes one or more various blood treatment devices, comprising the steps of: (a) switching by a controller one or more conduit switching valves provided at appropriate locations in said blood constituent treating circuit so as to create a local circuit in one part thereof; (b) circulating to said local circuit an initial circulating fluid of physiological saline fluid or an equivalent thereof; and (c) initiating by said controller a completing action according to visual evaluation by the operator; (d) said controller terminating one such set of steps (a), (b), and (c) before again performing another such set of steps (a), (b), and (c) to create another separate local circuit, said steps (a), (b), and (c) being repeatedly performed until substantially all of the air and other foreign matter in said blood treatment devices in said blood constituent treatment circuit has been purged and said blood constituent treatment circuit is substantially full only of said initial circulating fluid.

Further, according to the apparatus aspect of the present invention, these and other objects are more particularly and concretely accomplished by an apparatus for initially priming a blood constituent treatment circuit which includes one or more various blood treatment devices, comprising: (a) one or more conduit switching valves provided at appropriate locations in said blood constituent treating circuit, which are selectively switchable so as to establish one or more local circuits in one or more parts thereof; and (b) a means for controlling said one or more conduit switching valves so as to circulate to said local circuit an initial circulating fluid of

- 10 -

0181139

physiological saline fluid or an equivalent thereof, for initiating a completing action according to visual evaluation by the operator, for terminating one such local circuit establishment condition before again establishing another separate local circuit, and for repeatedly performing such local circuit establishment until substantially all of the air and other foreign matter in said blood treatment devices in said blood constituent treatment circuit has been purged and said blood constituent treatment circuit is substantially full only of said initial circulating fluid.

BRIEF DESCRIPTION OF THE DRAWINGS

The present invention will now be shown and described with reference to the preferred embodiment thereof, and with reference to the illustrative drawings, which however are all of them given purely for the purposes of explanation and exemplification only, and are none of them intended to be limitative of the scope of the present invention in any way. In the drawings, like parts and spaces and so on are denoted by like reference symbols in the various figures thereof; in the description, spatial terms are to be everywhere understood in terms of the relevant figure; and:

Fig. 1 is a frontal view showing a control panel incorporated in the apparatus of the present invention;

Fig. 2 is a schematic diagram showing various components of an exemplary blood constituent treatment circuit assembled on a panel of the apparatus of the present·invention;

Fig. 3 is a plan view showing the construction of an automatically actuatable conduit switching valve of which several are incorporated in said blood constituent treatment circuit;

Fig. 4 is a side view of said conduit switching valve with one clamp arm thereof omitted;

Fig. 5 is a plan view showing an alternative possible construction for said automatically actuatable conduit switching valve;

Fig. 6 is a side view of said alternative construction type conduit switching valve;

Figs. 7 through 18 are partial fluid circuit diagrams for explanation of the priming and purging process for said exemplary blood constituent treatment circuit of Fig. 2, according to the preferred embodiment of the method of the present invention;

Fig. 19 and Fig. 20 are partial fluid circuit diagrams showing the operation of said preferred embodiment during blood plasma recovery operation;

Fig. 21 is a perspective view of a prior art type of blood treatment machine;  and

Figs. 22 through 25 are partial fluid circuit diagrams for the operation of said prior art type blood treatment machine, similar to Figs. 2 and 7 through 20 relating to the present invention.

## DESCRIPTION OF THE PREFERRED EMBODIMENT

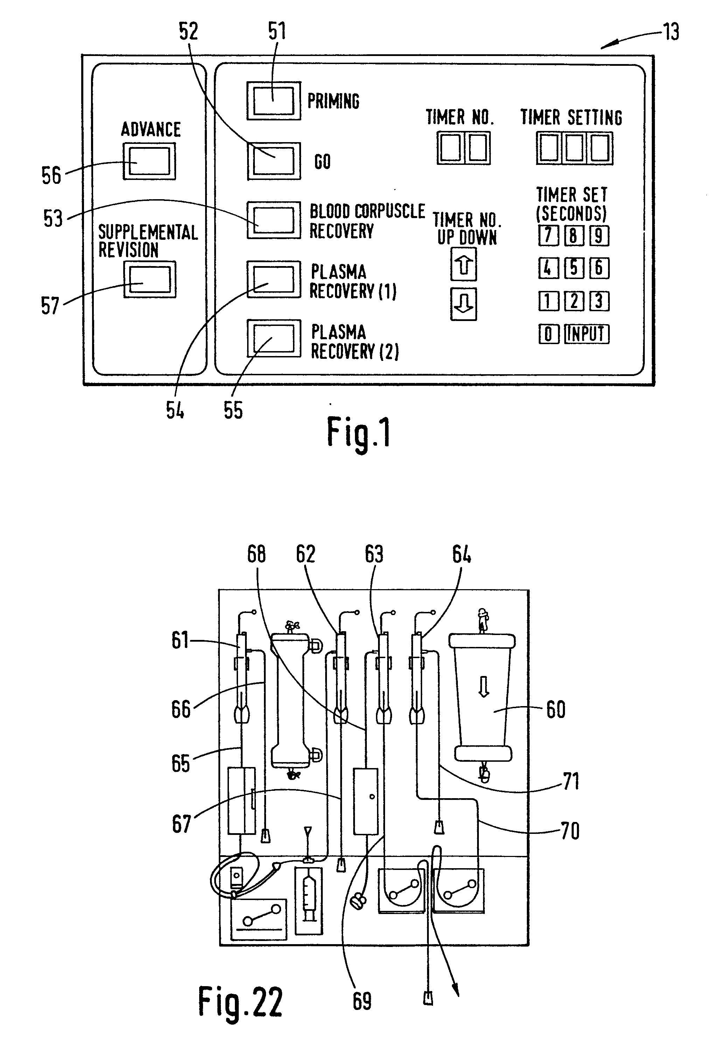

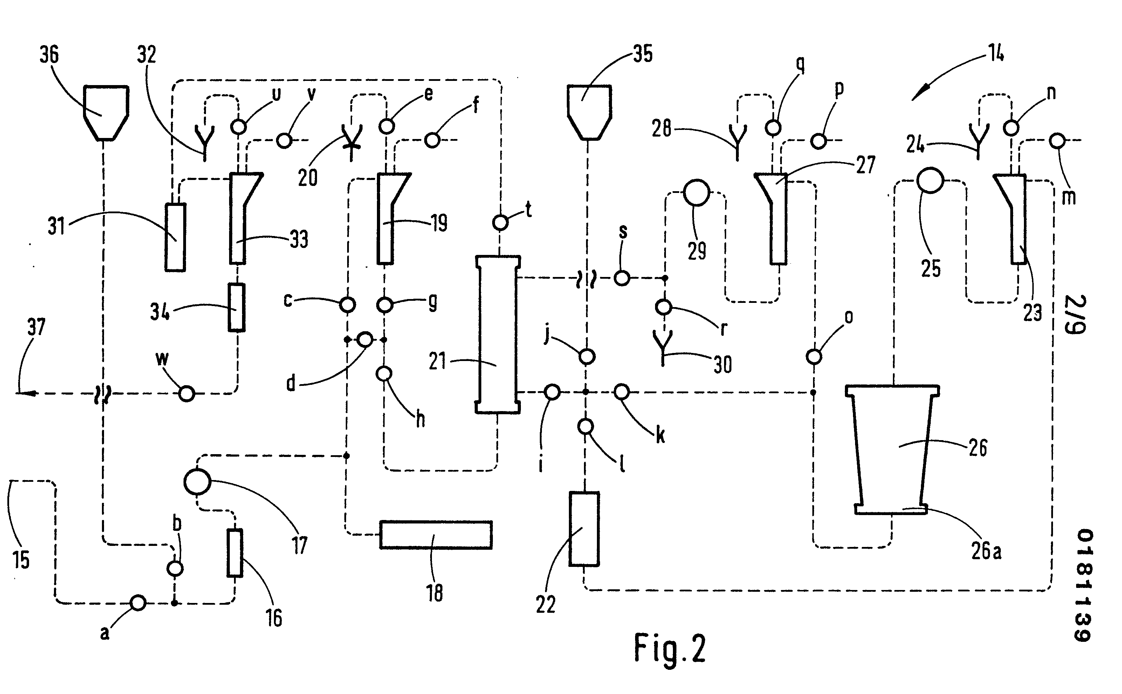

The present invention will now be described with reference to the preferred embodiment thereof, and with reference to the appended drawings. Fig. 1 is a frontal view showing a control panel 13 incorporated in the apparatus of the present invention. According to the apparatus and the method of the present invention, a controller, not particularly shown, controls the operation of various components of an assembled blood constituent treatment circuit 14, as exemplarily shown in Fig. 2, according to information input to said controller from the control panel 13. The constructional details of this controller, which typically is an at least partly electronic controller incorporating a microcomputer or the like, and the details of the programming thereof, will not be particularly discussed herein. This is because, based upon the disclosure and discussions of the functions of this controller set forth in this specification, various possible such structures will be relatively easily conceived of by a person of ordinary skill in the relevant art without undue experimentation. The control panel 13 supports the following push buttons: a priming push button 51, a go push button 52, a corpuscle recovery push button 53, two plasma recovery push buttons 54 and 55, an advance push button 56, and a supplemental revision push button 57. The functions of these push buttons will be outlined in the following. Further, the control panel 13 supports various control means and display means relating to the setting and monitoring of one or more timers, which will not be particularly described herein since, based upon the disclosure herein, various possibilities will be easily conceived of by a person of ordinary skill in the relevant art without undue experimentation.

Fig. 2 shows an exemplary set of devices included in an exemplary blood constituent treatment circuit of the preferred embodiment of the apparatus of the present invention which is set up on a setting up panel 38 (not particularly shown in this figure) like the setting up panel 2 of the previously described prior art; this example is however not intended to be limitative of the present invention, but is given for the purposes of description only. Now, the devices denoted by the various symbols will be briefly explained. The reference symbols "a" through "w" show the positions at which certain conduit switching valves to be described shortly are arranged and also denote said conduit switching valves; 15 is the starting portion of the shown exemplary blood constituent treatment circuit, and is connected to a suitable point in the circulatory system of a living human patient for taking out blood from the body of said patient for treatment thereof; 16 is a pressure detector for the extracted blood; 17 is a blood pump; 18 is a syringe type heparin pump; 19 is a second dripper; 20, 28, 24, 30, and 32 are waste fluid conduits; 21 is a blood plasma separator; 22 is a blood leakage detector; 23 is a fourth dripper; and 25 and 29 are both fluid supply pumps. Further, 26 is a blood plasma adsorber, at the outflow end of which is provided a membrane filter 26a; 27 is a third dripper; 31 is a heater for the returned blood; 33 is a first dripper; 34 is an air bubble detector; 35 is a second pack of physiological saline solution; 36 is a first pack of the same; and 37 is the end portion of the shown exemplary blood constituent treatment circuit, and is connected to another suitable point in the circulatory system of said patient for returning blood to the body of said patient after treatment thereof.

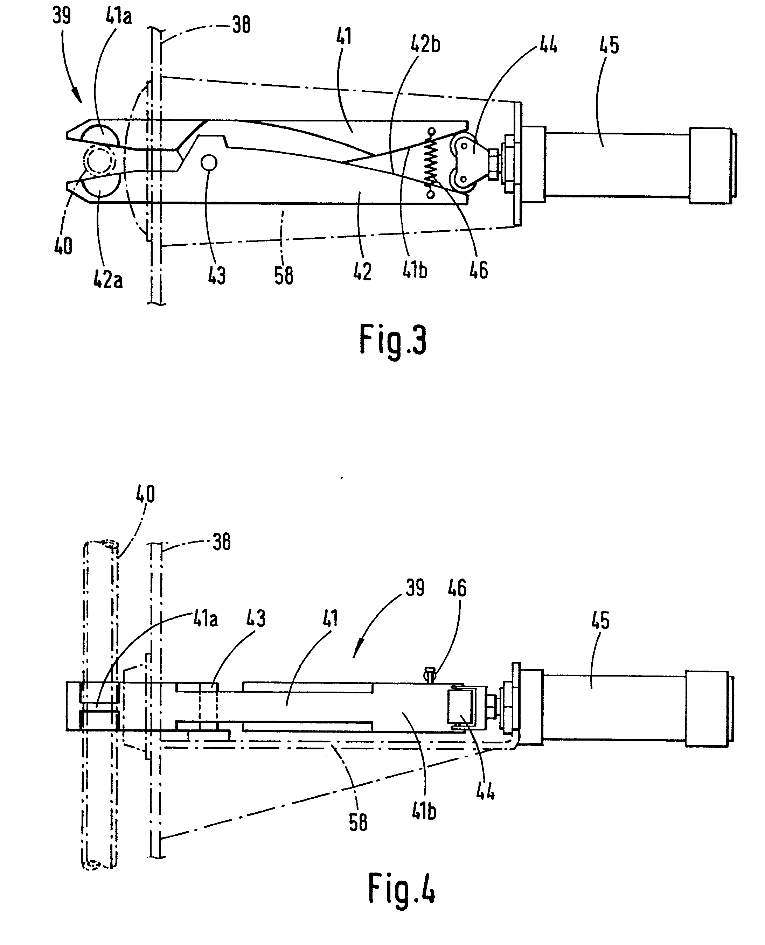

Each of the conduit switching valves "a" through "w" is mounted to the setting up panel 38, and is of a type which can selectively interrupt fluid flow through a fluid conduit to which it is fitted, according to a control signal which it receives from the aforementioned controller, not particularly shown. In the shown construction, each of these conduit switching valves is of a type which is selectively actuated by selective supply of compressed air thereto from said controller, which controls said compressed air supply by selective actuation of an air flow switch or the like not particularly shown. And it is convenient for these conduit switching valves to be so made that they can easily be fitted to and removed from conduits communication through which is to be controlled, according to the particular configuration required for the blood constituent treatment circuit, without otherwise disturbing said conduits. In Figs. 3 and 4 of the accompanying drawings, a first particular preferred structure for such a conduit switching valve 39 is shown; this valve is for application to a fluid conduit which has flexible walls and thus can be selectively squeezed shut.

In Fig. 3, a plan view of the conduit switching valve 39 is given. This valve 39 has two clamp arms 41 and 42 which are hinged around an axial shaft 43 in the manner of a pair of scissors or pliers; the axial shaft 43 projects sideways from a support 58 which is secured to the back surface of the setting up panel 38, as best seen in the side view given in Fig. 4 in which the clamp arm 42 is omitted. The one ends of said clamp arms 41 and 42 project through an aperture, not shown, formed in said setting up panel 38, and are formed with relatively thin compressing edges 41a and 42a for pinching and flattening the flexible fluid conduit. And the other ends of said clamp arms 41 and 42 are formed with smooth cam shapes 41b and 42b which oppose one another, and these ends of said clamp arms 41 and 42 are biased towards one another by a tension coil spring 46 fitted between them in a state of tension. The end of the support 58 remote from the setting up panel 38 is bent around, and to it is mounted an air cylinder 45, which is selectively supplied with compressed air as described above from said controller by means not particularly shown. And the end of the driven member of said air cylinder 45 is fitted with two rollers 44 which bear on the aforementioned cam shapes 41b and 42b.

This conduit switching valve functions as follows: it is so positioned on the setting up panel 38 that an appropriate one 40 of the flexible fluid conduits runs between the compressing edges 41a and 42a of the clamp arms 41 and 42. When no compressed air is supplied by the controller to the air cylinder 45, then the tension coil spring 46 pulls the aforementioned ends of the clamp arms 41 and 42 which have the cam shapes 41b and 42b formed on them towards one another, thus pushing by cam action the rollers 44 and the driven member of said air cylinder 45 in the rightward direction as seen in the figures, and ramming said driven member into the body of said air cylinder 45. In this state of the apparatus, shown in Fig. 3, the compressing edges 41a and 42a of said clamp arms 41 and 42 are somewhat separated from one another, so as not to pinch the fluid conduit 40 substantially. On the other hand, when a supply of compressed air is supplied by the controller to the air cylinder 45, then its said driven member is expelled from its said body and together with the rollers 44 is driven leftwards from the point of view of the figures, and, against the biasing action of the tension coil spring 46 which is overcome, by the cam action of the rollers 44 sliding upon the cam shapes 41b and 42b, said ends of the clamp arms 41 and 42 which have said cam shapes 41b and 42b formed on them are pushed away from one another. In this state of the apparatus, not particularly shown in any of the figures, the compressing edges 41a and 42a of said clamp arms 41 and 42 are strongly brought together towards one another, so as strongly to pinch the fluid conduit 40, and due to the flexibility of its walls said fluid conduit 40 is so narrowed down as to substantially interrupt all fluid flow therethrough. In other words, the flow of blood or of a blood constituent, or of physiological saline solution, through said fluid conduit 40 is interrupted.

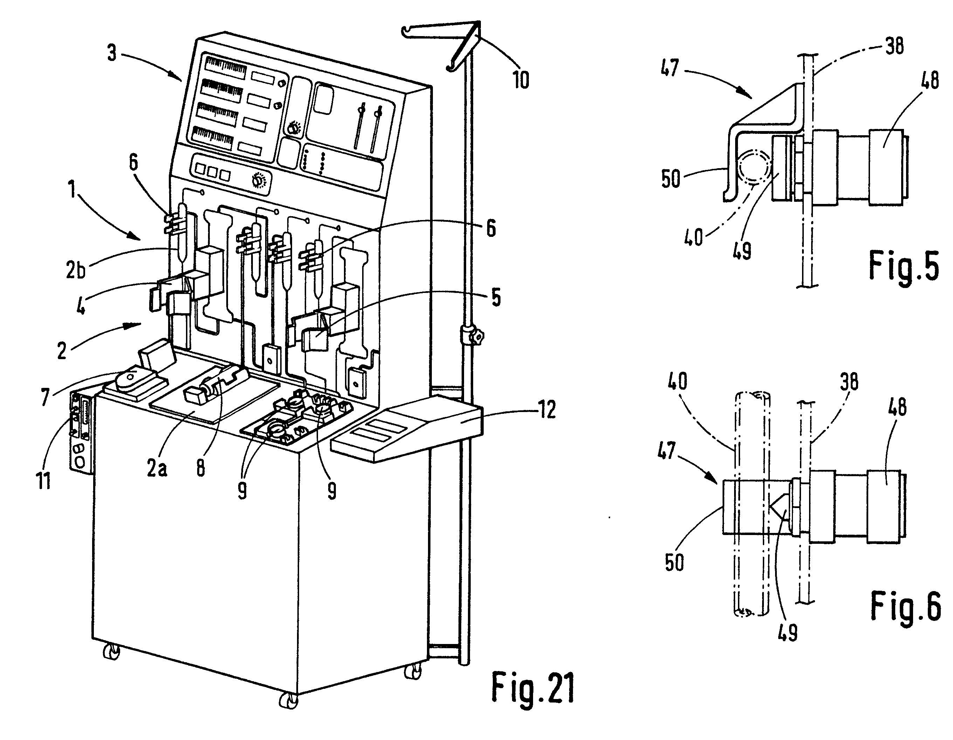

In Fig. 5, a plan view of another possible preferred construction for said conduit switching valve, this time denoted as 47, is given. This valve 47 has a compression piece 49 formed with a relatively thin compression edge portion and with a cross

- 18 -

0181139

sectional shape in which said compression edge portion forms a ridge, as best seen in the side view of the conduit switching valve 47 given in Fig. 6. This compression piece 49 is directly fitted on the end of the driven member of the air cylinder 48 (and may be in fact constituted by its end portion), and said air cylinder 48 is, in this variant construction, directly mounted on the back surface of the setting up panel 38. And a backing member 50 is secured to the front surface of said setting up panel 38, a bent round portion thereof opposing the compression edge portion of the compression piece 49 with a certain gap being left between them. The flexible walled fluid conduit 40 is led so as to pass between said bent round portion of the backing member 50 and said compression edge portion of the compression piece 49, as best seen in Fig. 5. Accordingly, when no compressed air is supplied by the controller to the air cylinder 48, then the driven member of said air cylinder 48 is retracted in the rightward direction as seen in the figures, by the elasticity of the walls of the conduit 40 or by a biasing means not particularly shown in the figures, and the compressing edge of the compression piece 49 and the bent round portion of the backing member 50 are somewhat separated from one another, so as not to pinch the fluid conduit 40 substantially. On the other hand, when a supply of compressed air is supplied by the

- 19 -

0181139

controller to the air cylinder 48, then its said driven member is expelled from its said body and is driven leftwards from the point of view of the figures, and, against the elasticity of the walls of the conduit 40 or the action of the aforementioned biasing means, not shown, which is overcome, said compressing edge of said compression piece 49 and said bent round portion of the backing member 50 are strongly brought together towards one another, so as strongly to pinch the fluid conduit 40, and, similarly to the previous case, due to the flexibility of its walls, said fluid conduit 40 is thereby so narrowed down as to substantially interrupt all fluid flow therethrough. In other words, again, the flow of blood or of a blood constituent, or of physiological saline solution, through said fluid conduit 40 is interrupted.

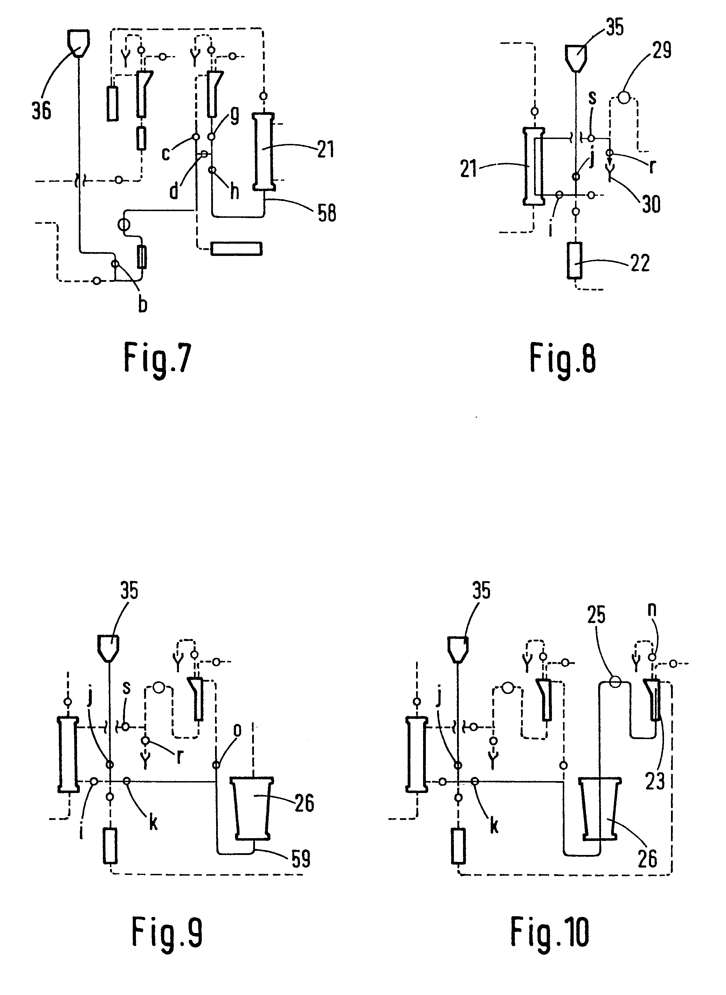

In the following, the method of the present invention will be explained, as practiced by the apparatus of the present invention disclosed above. This method comprises thirteen steps. In Figs. 7 through 18, portions of the blood constituent treatment circuit of Fig. 2 are shown as connected up into the local circuits formed in each of said thirteen steps.

First, before commencing the practice of the method of the present invention, a process of prepreparation is performed. In this process, there are installed, on the setting up panel 38 of the apparatus of the present invention described above, the first through the fourth drippers 33, 19, 27, and 23, the blood plasma separator 21, and the blood plasma adsorber 26, and further all of these devices other than the blood plasma separator 21 and the blood plasma adsorber 26 are connected by the fluid supply conduits 40. Next, as indicated in Figs. 3 and 4, each of the fluid supply conduits 40 is fitted in between the two compressing edges 41a and 42a of the clamp arms 41 and 42 of an appropriate one of the conduit switching valves 39 provided on said setting up panel 38. Further, on the control panel 13 shown in Fig. 1, the correct prescribed time for each step of the operation is set (this may be in fact done by extracting such data from a memory, in which said data has been stored in advance), and this completes the prepreparation process.

When the operator presses the priming push button 51 on the control panel 13, this starts the apparatus of the present invention off on the initial priming stage thereof. At this time point, all of the conduit switching valves "a" through "w" of the circuit diagram of Fig. 2 are in the closed state, with air pressure being supplied to their actuating cylinders by the controller. Next, the operator presses the advance push button 56, to commence the first step of operation. In this first step, the controller, as shown in Fig. 7, opens only the conduit switching valves "b" and "c". Then, at the time point when the physiological saline solution from the first pack 36 thereof passes the conduit switching valve "c", immediately said conduit switching valve "c" is closed and the conduit switching valves "d" and "g" are opened. Further, at the time point when said physiological saline solution passes the conduit switching valve "g", immediately the conduit switching valves "b", "d", and "h" are opened, and said physiological saline solution is directed to the unconnected end conduit portion 58. At this time point, the controller closes the conduit switching valve "h" and stops operation, whereupon it informs the operator, via appropriate warning means such as a buzzer, a lamp, or the like, not particularly shown and per se conventional, that the end of the first step has arrived. After the operator has verified that none of the conduit switching valves has mistakenly moved, he or she repeatedly pushes the supplemental revision push button 57 on the control panel 13 several times. Accordingly, the conduit switching valve "h" is caused to open and close several times, and in due course the physiological saline solution begins to overflow from said unconnected end conduit portion 58. Then, with the physiological saline solution in a continuously overflowing condition from said unconnected end conduit portion 58, said unconnected end conduit portion 58 is connected to the inflow side of the blood plasma separator 21, and this completes the first step of the process.

Next, the operator again presses the advance push button 56, to commence the second step of operation. In this second step, the controller, as shown in Fig. 8, closes the conduit switching valves "b", "d", and "h" which were opened during the first step, and concurrently opens the conduit switching valves "j", "i", "s", and "r". In this second step, therefore, the physiological saline solution from the second pack 35 thereof is led through the interior of the blood plasma separator 21, and into the waste fluid conduit 30, thereby removing the air from and cleansing said blood plasma separator 21. Then, after the preset prescribed time period has elapsed, the controller stops this operation, in a manner similar to its operation at the end of the first step. After the operator has verified that none of the conduit switching valves has mistakenly moved, he or she pushes the advance push button 56 on the control panel 13, to terminate this second step and to commence the third step.

In this third step, the controller, as shown in Fig. 9, closes the conduit switching valves "i", "s", and "r" which were opened during the second step, and concurrently opens the conduit switching valves "k" and "o". In this third step, a process essentially akin to that performed in the first step for the connection of the blood plasma separator 21 as shown in Fig. 7 and described above is conducted, with the unconnected end conduit portion 59 of the fluid conduit being connected to the outflow side of the blood plasma adsorber 26. As will by now be clear from the above, the operation of each of the remaining steps is essentially similar to the procedures according to which the above described first through third steps are performed, and hence the description thereof given herein will be relatively brief.

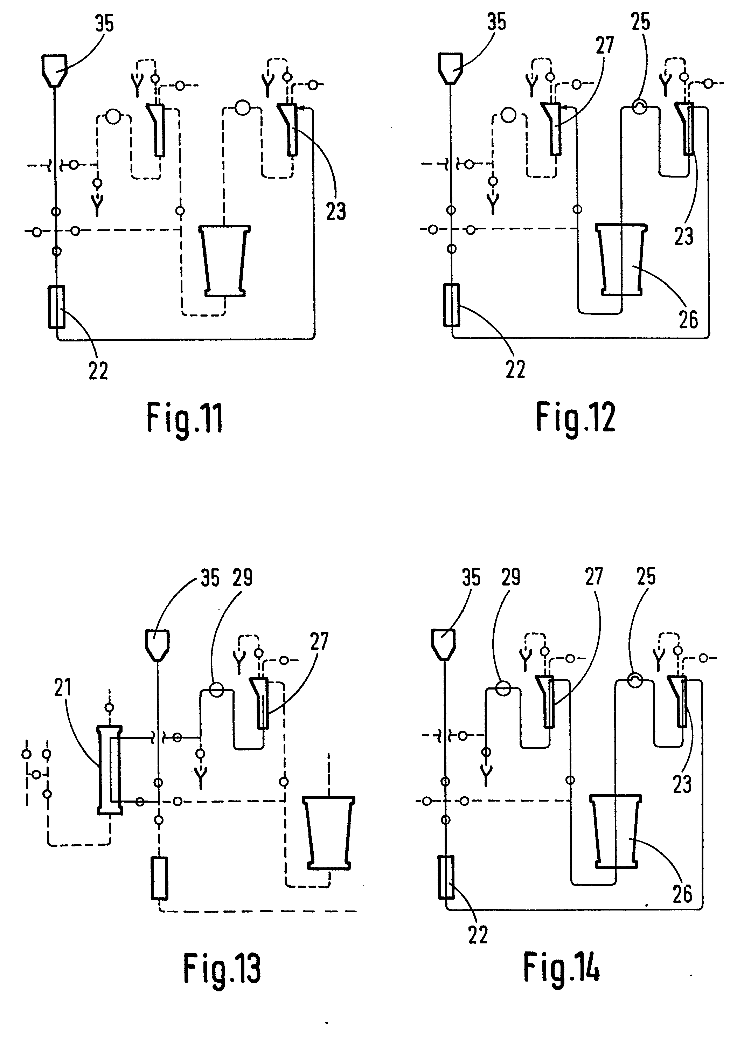

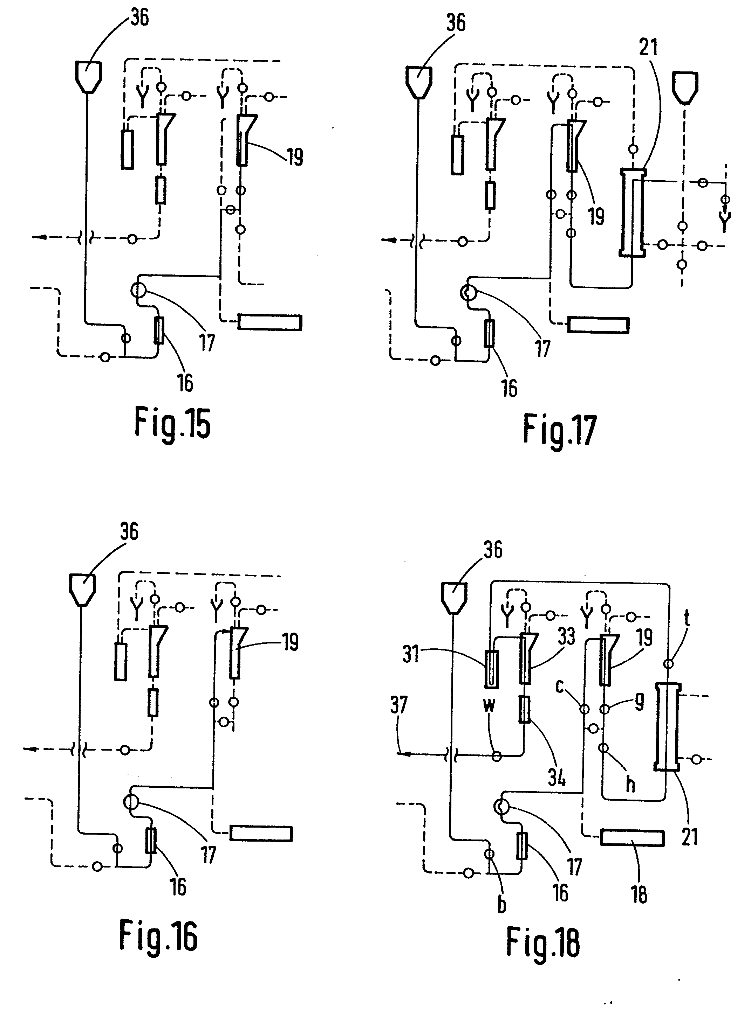

In the third step, the controller, as shown in Fig. 10, opens the conduit switching valve "n", thus expelling the air and foreign matter in the blood plasma adsorber 26 to the fourth dripper 23. Fig. 11 shows the operation of the fifth step, in which the fluid flowing from the second physiological saline pack 35 passes through the blood leakage detector 22, and reaches the fourth dripper 23, and accordingly these units are primed, purged of air, and cleansed. Fig. 12 shows the operation of the sixth step, in which the local circuits up to the fifth step as described above are further extended to reach the third dripper 27. Fig. 13 shows the operation of the seventh step, in which the physiological saline solution is circulated in reverse through the fluid conduits, passing through the blood plasma separator 21, to reach the third dripper 27, in order to prime, purge air from, and clean said fluid conduits. Fig. 14 shows the operation of the eighth step, in which, effectively, an adsorption circuit for low density lipoprotein is established, making individual connection with the second physiological saline solution pack 35, the blood leakage detector 22, the fourth dripper 23, the fluid supply pump 25, the blood plasma adsorber 26, the third dripper 27, and the fluid supply pump 29; and cleansing is effected through the interior of said low density lipoprotein adsorption circuit. Moreover, although it is omitted in the figures, the local circuit established according to this eighth step continues to be in effect through the subsequent ninth through thirteenth steps. Fig. 15 shows the ninth step, in which the air in the end portion of the fluid supply conduit connected to the outflow side of the second dripper 19 is expelled therethrough to said second dripper 19. Fig. 16 shows the tenth step, in which the air in the end portion of the fluid supply conduit connected to the inflow side of the second dripper 19 is expelled therethrough to said second dripper 19. Fig. 17 shows the eleventh step, in which the physiological saline solution is circulated through the proper circuit, passing through the second dripper 19 and reaching the blood plasma separator 21, whereupon the interior of the fluid supply conduit connected to the upper side connection portion (the blood plasma mixing in portion) of said blood plasma separator 21 is concurrently cleansed. Fig. 18 shows the operation of the twelfth step, in which, effectively, there is established a blood plasma separating circuit communicating individually with the first physiological saline solution pack 36, the blood collecting pressure detector 16, the blood pump 17, the second dripper 19, the blood plasma separator 21, the blood heater 31, the first dripper 33, and the air bubble detector 34, and cleansing is accomplished through the interior of said blood plasma separating circuit.

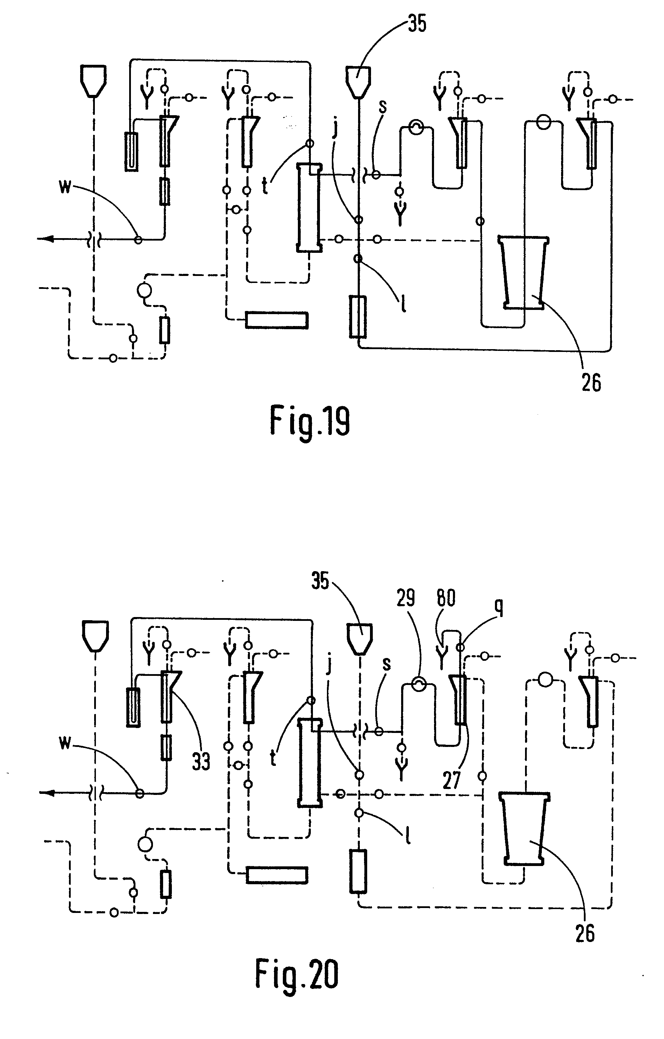

Moreover, since, as shown in Figs. 3 and 4 and described above, the controller is provided for controlling with selectable timing the driving of the conduit switching valves 39 which are provided on the setting up panel 38 of the apparatus of the present invention, thereby blood constituent treatment therapy is made available, and further in the final phase of this therapy the recovery of blood constituents such as blood corpuscles, blood plasma, and the like which remain in the circuit is also made possible. In detail, when the blood corpuscle recovery button 53 on the control panel 13 shown in Fig. 1 is pushed by the operator, then the same circuit as that in the twelfth step of the initial priming operation described above (see Fig. 18) is established. That is, as shown in Fig. 18, the conduit switching valves "b", "c", "g", "h", "t", and "w" are opened, and the physiological saline solution from the first pack 36 thereof pushes and circulates the blood and the blood corpuscles remaining in the blood plasma separator 21 and the fluid supply conduits into the body of the patient. Furthermore, in this case, since there is still blood in the circuit leading to the blood plasma separator 21 that has yet to be separated, it is desirable that the heparin pump 18 should be driven intermittently. When the blood plasma recovery (1) button 54 on the control panel 13 shown in Fig. 1 is pushed by the operator, then as shown in Fig. 19 the conduit switching valves "j", "l", "o", "s", "t", and "w" are opened by the controller, and the physiological saline solution from the first pack 36 thereof pushes and circulates the blood plasma remaining in the blood plasma adsorber 26 and the fluid supply conduits into the body of the patient. Further, after it has been verified that the blood plasma in said blood plasma adsorber 26 has for the most part been pushed and circulated out by the physiological saline solution, then the blood plasma recovery (2) button 55 on the control panel 13 shown in Fig. 1 is pushed by the operator. At this time, the conduit switching valves "j", "l", and "o", which up

- 27 -

0181139

till now were open, are closed by the controller, and thus the physiological saline solution in the second pack 35 thereof is isolated. Then, the controller opens the conduit switching valve "q" on the upper portion of the third dripper 27 again. Accordingly, air is sucked in from the release end portion of the fluid supply conduit by the fluid supply pump 29 which is in constant operation, and this air pushes out the remaining blood plasma in the circuit. However, since there is the danger in this operation that air will be introduced into the circulatory system of the patient, it is desirable that this operation should be stopped early on, at the point when the air bubbles in the blood plasma passing through the fluid supply conduit arrive at the first dripper 33.

In this manner, the apparatus according to the shown preferred embodiment of the present invention may be utilized for estabishing a great number and variety of different operational modes, according to the appropriate switching over of the conduit switching valves "a" through "w" arranged on its setting up panel 38. Of course, it is possible to set up other types of circuit for other purposes than the exemplarily shown one of adsorption removal of low density lipoprotein from blood plasma.

Thus, as will be understood from the above explanations, the initial priming of the blood constituent treatment circuit according to the method and the apparatus of the present invention differ from the prior art detailed above in that the controller fulfils the functions of the operator in effecting each prescribed action, and therefore there is no need for the operator to go through the sequential steps of operation while clutching an operation manual in his or her hand. Moreover, because such matters are attended to by the controller, there is no likelihood of mistakes occurring in operational procedure during this initial priming and purging process, nor of omission of intermediate steps. Further, since the controller suspends the operation of each step before it is completely finished, thereby, when the next action is to be proceeded to, since the operator must issue a starting command by his or her own human agency before the next action can be commenced by the controller, at each step operator verification is prompted for and then awaited. Thus, it is seen that the method and the apparatus of the present invention are epoch making, in that the work required from the operator is extremely easy, and, even in the case of an operator who is unfamiliar with the apparatus of the present invention, it is possible to establish and enjoy proper, reliable, swift, and safe initial priming and purging operation.

Although the present invention has been shown and described with reference to the preferred embodiment thereof, and in terms of the illustrative drawings, it should not be considered as being limited thereby, since many alterations could be made in the perhaps purely fortuitous details of the embodiment and the drawings, but as being defined solely by the scope of the appended claims, which follow.

CLAIMS

1.   A method for initially priming a blood constituent treatment circuit which includes one or more various blood treatment devices, comprising the steps of:

(a)   switching by a controller one or more conduit switching valves provided at appropriate locations in said blood constituent treating circuit so as to create a local circuit in one part thereof;

(b)   circulating to said local circuit an initial circulating fluid of physiological saline fluid or an equivalent thereof;

and

(c)   initiating by said controller a completing action according to visual evaluation by the operator;

(d)   said controller terminating one such set of steps (a), (b), and (c) before again performing another such set of steps (a), (b), and (c) to create another
separate local circuit, said steps (a), (b), and (c)
being repeatedly performed until substantially all of
the air and other foreign matter in said blood
treatment devices in said blood constituent treatment
circuit has been purged and said blood constituent
treatment circuit is substantially full only of said
initial circulating fluid.

2.    An apparatus for initially priming a blood
constituent treatment circuit which includes one or
more various blood treatment devices, comprising:

(a)    one or more conduit switching valves provided at
appropriate locations in said blood constituent
treating circuit, which are selectively switchable so
as to establish one or more local circuits in one or
more parts thereof;   and

(b)    a means for controlling said one or more conduit
switching valves so as to circulate to said local
circuit an initial circulating fluid of physiological
saline fluid or an equivalent thereof, for initiating a completing action according to visual evaluation by the operator, for terminating one such local circuit establishment condition before again establishing another separate local circuit, and for repeatedly performing such local circuit establishment until substantially all of the air and other foreign matter in said blood treatment devices in said blood constituent treatment circuit has been purged and said blood constituent treatment circuit is substantially full only of said initial circulating fluid.

0181139

Fig.1

Fig.22

Fig.2

2/9

0181139

0181139

Fig.3

Fig.4

Fig.21

Fig.5

Fig.6

0181139

Fig.7

Fig.8

Fig.9

Fig.10

Fig.11

Fig.12

Fig.13

Fig.14

Fig.15

Fig.17

Fig.16

Fig.18

0181139

Fig.19

Fig.20

0181139

Fig.23

Fig.24

Fig.25